(19) Europäisches Patentamt
European Patent Office
Office européen des brevets

(11) **EP 3 213 670 A1**

(12) **EUROPEAN PATENT APPLICATION**

(43) Date of publication:
06.09.2017 Bulletin 2017/36

(51) Int Cl.:
*A61B 3/12* (2006.01)  *A61F 9/008* (2006.01)

(21) Application number: 17158832.0

(22) Date of filing: 02.03.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 02.03.2016 JP 2016040538

(71) Applicant: Nidek co., Ltd.
Gamagori
Aichi (JP)

(72) Inventors:
• FURUUCHI, Yasuhiro
Gamagori, Aichi (JP)
• HANEBUCHI, Masaaki
Gamagori, Aichi (JP)

(74) Representative: Hoefer & Partner Patentanwälte mbB
Pilgersheimer Straße 20
81543 München (DE)

(54) **OPHTHALMIC LASER TREATMENT DEVICE, OPHTHALMIC LASER TREATMENT SYSTEM, AND LASER IRRADIATION PROGRAM**

(57) An ophthalmic laser treatment device includes an irradiation unit that irradiates a patient's eye with laser treatment light and a control unit that controls the irradiation unit. The control unit acquires a motion contrast acquired by an OCT unit that detects an OCT signal of measurement light reflected from the patients eye and reference light corresponding to the measurement light, acquires irradiation target information based on the motion contrast, and controls the irradiation unit so as to irradiate the patient's eye with the laser light, based on the irradiation target information.

*FIG.1*

EP 3 213 670 A1

**Description**

BACKGROUND

The present disclosure relates to an ophthalmic laser treatment device, an ophthalmic laser treatment system, and a laser irradiation program which are used in treating a patient's eye by irradiating the patient's eye with laser light.
For example, as a laser treatment device in the related art, a laser treatment device is known which treats a patient's eye by irradiating tissues (for example, a fundus) of the patient's eye with laser treatment light (refer to JP-A-2010-148635). In a case of using this laser treatment device, an operator observes a fundus front image by using a slit lamp and a fundus camera, and irradiates a treatment target of the eye with the laser light.

SUMMARY

However, according to the fundus front image in the related art, a proper position for irradiating a blood vessel of a fundus with the laser light is not recognized.
An aspect of the present invention is made in view of the above-described circumstances, and a technical object thereof is to provide an ophthalmic laser treatment device, an ophthalmic laser treatment system, and a laser irradiation program which can irradiate a suitable irradiation position with laser light.
In order to solve the above-described problem, an aspect of the present disclosure includes the following configurations.

(1) An ophthalmic laser treatment device comprising:

an irradiation unit configured to irradiate a patient's eye with laser treatment light; and
control means for:

acquiring a motion contrast acquired by an OCT unit configured to detect an OCT signal of measurement light reflected from the patient's eye and reference light corresponding to the measurement light;
acquire irradiation target information based on the motion contrast; and
control the irradiation unit to irradiate the patient's eye with the laser light based on the irradiation target information.

(2) The ophthalmic laser treatment device according to (1) further comprising:

an image capturing unit configured to capture a fundus front image of the patient's eye,
wherein the control means aligns an image of the motion contrast and the fundus front image with each other, and irradiates an irradiation target of which the irradiation target information is associated with the fundus front image, with the laser light.

(3) The ophthalmic laser treatment device according to (2), wherein the control means detects displacement of the irradiation target which occurs due to a motion of the patient's eye, from the fundus front images which are frequently captured by the image capturing unit, and causes an irradiation position of the laser light to track the irradiation target based on the detected displacement.

(4) The ophthalmic laser treatment device according to (1), wherein the control means controls a focal position of the laser light, based on the irradiation target information.

(5) The ophthalmic laser treatment device according to (1),
wherein the control means acquires, from the OCT unit, the motion contrasts in a region including at least an irradiation position of the laser light used for irradiation based on the irradiation target information, and compares the motion contrast acquired before the laser light irradiation and the motion contrast acquired after the laser light irradiation with each other.

(6) An ophthalmic laser treatment system comprising:

an ophthalmic laser treatment device configured to irradiate a patient's eye with laser treatment light; and
an OCT device configured to detect an OCT signal of measurement light reflected from the patient's eye and reference light corresponding to the measurement light,

wherein the OCT device calculates a motion contrast, based on the OCT signal, and
wherein the ophthalmic laser treatment device acquires irradiation target information based on the motion contrast, and irradiates the patient's eye with the laser light, based on the irradiation target information.

(7) A method of controlling an ophthalmic laser treatment device, the method comprising steps of:

acquiring a motion contrast acquired by an OCT unit that detects an OCT signal of measurement light reflected from a patient's eye and reference light corresponding to the measurement light;
acquiring irradiation target information based on the motion contrast; and
irradiating the patient's eye with laser treatment light based on the irradiation target information.

BRIEF DESCRIPTION OF THE DRAWINGS

Fig. 1 is a schematic configuration diagram for describing a configuration of a laser treatment device according to the present embodiment.
Fig. 2 is a flowchart illustrating a control operation of the laser treatment device according to the present embodiment.
Fig. 3 is a view for describing ocular fundus scanning of an OCT unit.
Fig. 4 is a view illustrating an example of a motion contrast image and a motion contrast front image.
Fig. 5 is a view illustrating an example of a fundus front image and the motion contrast image.
Fig. 6 is a view for describing setting of a laser irradiation position in a surface direction of a fundus.
Figs. 7A and 7B are views for describing setting of a laser focusing position in a depth direction of the fundus.
Fig. 8 is a view for describing image capturing of the motion contrast image obtained after laser irradiation.

DESCRIPTION OF ILLUSTRATIVE EMBODIMENTS

Hereinafter, an embodiment according to the present disclosure will be briefly described. An ophthalmic laser treatment device (for example, a laser treatment device 1) according to the present embodiment mainly includes an irradiation unit and a control unit (for example, a control unit 70). For example, the irradiation unit irradiates a patient's eye with laser treatment light. For example, the irradiation unit includes a laser treatment light source (for example, a laser light source 401) and a scanning unit (for example, a scanning unit 408) which scans the patient's eye with the laser light emitted from the light source. For example, the control unit controls the irradiation unit.

For example, the control unit acquires a motion contrast. For example, the motion contrast is acquired by an OCT unit (OCT unit 100). For example, the OCT unit detects an OCT signal of measurement light reflected from the patient's eye and reference light corresponding to the measurement light. For example, the motion contrast may be information obtained by recognizing a motion of an object (for example, blood flow or change in tissues).

For example, the control unit acquires irradiation target information based on the motion contrast. For example, the irradiation target information may be position information of a blood vessel, position information of a lesion, or position information of an affected area. For example, the irradiation target information may be position information designated by an operator. For example, the control unit 70 controls the irradiation unit so as to irradiate an irradiation target with the laser light, based on the irradiation target information. In this manner, the present laser treatment device can set a suitable irradiation position of the laser light by using blood vessel information acquired using the motion contrast.

The present laser treatment device may include an image capturing unit (for example, an observation system 200). For example, the image capturing unit captures a fundus front image of the patient's eye. For example, the image capturing unit may be a scanning laser ophthalmoscope (SLO), a fundus camera, and a slit lamp. In this case, the control unit may align a motion contrast image and the fundus front image with each other so that the irradiation target whose irradiation target information is associated with the fundus front image is irradiated with the laser light.

The control unit may cause the image capturing unit to detect displacement of the irradiation target, which occurs due to the motion of the patient's eye, from the frequently captured ocular fundus front images, and may follow the irradiation position of the laser light, based on the displacement. In this manner, in a case where the motion contrast is less likely to be acquired on a real time basis, the control unit can perform a tracking process on the image captured by the image capturing unit on the real time basis.

The image of the motion contrast may be a motion contrast front image. For example, the image may be an En face image of the motion contrast. Here, an En face may be a plane horizontal to a fundus surface or two-dimensional horizontal tomographic plane of a fundus.

For example, the control unit may correct the distortion of the image between the motion contrast front image and the fundus front image. For example, the control unit may detect distortion information of the image between the motion contrast front image and the fundus front image, and may correct the distortion of at least any one image of the both images, based on the distortion information. In this manner, the control unit may be likely to align both images with each other. The control unit may apply the distortion information of the motion contrast image to all of the motion contrasts acquired three-dimensionally.

The control unit may control a focal position of the laser light, based on the irradiation target information. For example, the control unit may adjust the focal position (focal length) of the laser light, based on position information in a depth direction of the irradiation target. In this manner, the present laser treatment device can accurately irradiate the affected area with the laser light.

The control unit may acquire each motion contrast before and after laser light irradiation. In this case, for example, the control unit acquires the motion contrast in a region including at least the irradiation position of the laser light used for irradiation based on the irradiation target information. Then, the control unit may compare the motion contrast obtained before the laser light irradiation and the motion contrast obtained after the laser light irradiation with each other. For example, the control unit 70 may calculate a difference between both of these. In this manner, the present laser treatment device can acquire a change in a treatment site before and after the laser light irradiation.

The ophthalmic laser treatment device may configure an OCT device and an ophthalmic laser treatment system. In this case, for example, the ophthalmic laser treatment device acquires the irradiation target information based on the motion contrast acquired by the OCT device, and irradiates the irradiation target with the laser light, based on the irradiation target information. As a matter of course, the present laser treatment device may include the OCT unit.

The control unit may execute a laser irradiation program stored in a storage unit (for example, a ROM 72, a RAM 73, a storage unit 74, and the like). For example, the laser irradiation program includes a first acquisition step, a second acquisition step, and an irradiation step. For example, the first step is a step of acquiring the motion contrast acquired by the OCT unit which detects the OCT signal of the measurement light reflected from the patient's eye and the reference light corresponding to the measurement light. The second step is a step of acquiring the irradiation target information based on the motion contrast. The irradiation step is a step of irradiating the patient's eye with the laser treatment light, based on the irradiation target information.

Embodiment

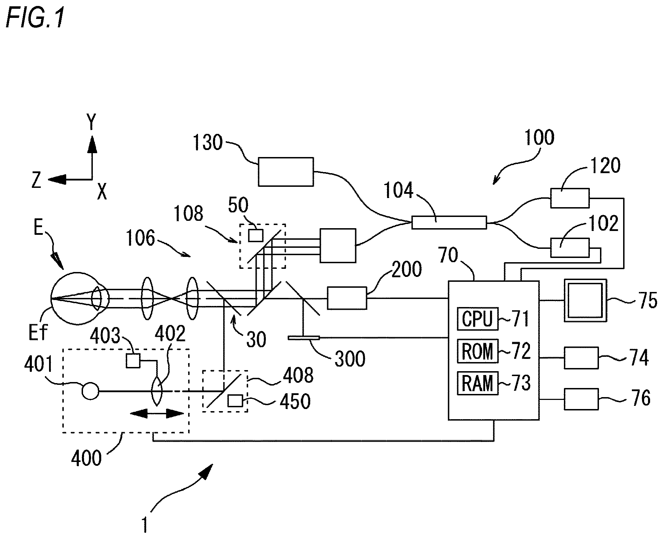

Hereinafter, an embodiment according to the present disclosure will be described. Fig. 1 is a schematic configuration diagram for describing a configuration of the laser treatment device according to the present embodiment. In the present embodiment, description will be made on the assumption that an axial direction of the patient's eye E is a Z-direction, a horizontal direction is an X-direction, and a vertical direction is a Y-direction. It may be considered that a surface direction of ocular fundus is an XY-direction.

The laser treatment device 1 treats a patient's eye E by irradiating a fundus Ef with the laser light. For example, the laser treatment device 1 includes the OCT unit 100, a laser unit 400, an observation system 200, a fixation guide unit 300, and the control unit 70.

OCT Unit

For example, the OCT unit 100 is an optical system for capturing a tomographic image of the fundus Ef of the patient's eye E. For example, the OCT unit 100 detects an interference state between the measurement light reflected from the fundus Ef and the reference light corresponding to the measurement light. The OCT unit 100 may adopt a configuration of so called optical coherence tomography (OCT). For example, the OCT unit 100 captures the tomographic image of the patient's eye E. For example, the OCT unit 100 includes a measurement light source 102, a coupler (beam splitter) 104, a scanning unit (for example, an optical scanner) 108, an objective optical system 106, a detector (for example, a light receiving element) 120, and a reference optical system 130. The objective optical system 106 may also serve as the laser unit 400 (to be described later).

The OCT unit 100 causes a coupler (beam splitter) 104 to split the light emitted from the measurement light source 102 into the measurement light (sample light) and the reference light. The OCT unit 100 guides the measurement light to the fundus Ef of the eye E via the scanning unit 108 and the objective optical system 106, and guides the reference light to the reference optical system 130. Thereafter, the OCT unit 100 causes a detector (light receiving element) 120 to receive interference light obtained by combining the measurement light reflected from the fundus Ef and the reference light with each other.

The detector 120 detects an interference state between the measurement light and the reference light. In a case of the Fourier domain OCT, spectral density of the interference light is detected by the detector 120, and a depth profile (A-scan signal) in a predetermined range is acquired by performing Fourier transformation on spectral intensity data. For example, spectral-domain OCT (SD-OCT) and swept-source OCT (SS-OCT) may be employed. In addition, time-domain OCT (TD-OCT) may also be employed.

In a case of the SD-OCT, a low coherent light source (broadband light source) is used as the light source 102. A spectroscopic optical system (spectrometer) for dispersing the interference light into each frequency component (each wavelength component) is disposed in the detector 120. For example, the spectrometer includes a diffraction grating and a line sensor.

In a case of the SS-OCT, a wavelength scanning-type light source (wavelength variable light source) for changing an emission wavelength very quickly is used as the light source 102. For example, a single light receiving element is disposed as the detector 120. For example, the light source 102 is configured to include a light source, a fiber ring resonator, and a wavelength selection filter. For example, the wavelength selection filter includes a combination between the diffraction grating and a polygon mirror or a Faby-Perot etalon.

The light emitted from the light source 102 is split into a measurement light beam and a reference light beam by the coupler 104. The measurement light beam is emitted into the air after being transmitted through an optical fiber. The light beam is emitted to the fundus Ef via the scanning unit 108 and the objective optical system 106. The light reflected from the fundus Ef returns to the optical fiber through the same optical path.

For example, the scanning unit 108 scans the fundus Ef with the measurement light in the XY-direction (transverse direction). For example, the scanning unit 108 is disposed at a position substantially conjugate with a pupil. For example, the scanning unit 108 includes two galvanometer mirrors, and a reflection angle thereof is optionally adjusted by a drive mechanism 50.

In this manner, a reflection (traveling) direction the light beam emitted from the light source 102 is changed, and the light beam is used for scanning the fundus Ef in an optional direction. In this manner, an imaging position on the fundus Ef is changed. The scanning unit 108 may adopt any configuration as long as the light is deflected. For example, in addition to a reflection mirror (galvano mirror, polygon mirror, or resonant scanner), an acousto optical modulator (AOM) for changing the traveling (deflection) direction of the light is used.

The reference optical system 130 generates the reference light combined with the reflected light acquired by the reflection of the measurement light reflected from the fundus Ef. The reference optical system 130 may be a Michelson type or a Mach-Zehnder type. For example, the reference optical system 130 is formed from a reflection optical system (for example, a reference mirror). The light from the coupler 104 is reflected by the reflection optical system, is caused to return to the coupler 104 again, and is guided to the detector 120. As another example, the reference optical system 130 is formed from a transmission optical system (for example, an optical fiber). The light from the coupler 104 is not caused to return to the coupler 104, is transmitted through the transmission optical system, and is guided to the detector 120.

The reference optical system 130 has a configuration in which an optical path length difference between the measurement light and the reference light is changed by moving an optical member in a reference light path. For example, the reference mirror is moved in an optical axis direction. The configuration for changing the optical path length difference may be disposed in a measurement light path of the objective optical system 106. The OCT unit 100 may refer to JP-A-2008-29467.

Observation System

For example, the observation system 200 is provided in order to obtain a fundus front image of the fundus Ef. The observation system 200 may have a configuration of a so called scanning laser ophthalmoscope (SLO). For example, the observation system 200 may include an optical scanner and a light receiving element. For example, the optical scanner may two-dimensionally scan the Fundus Ef with the measurement light (for example, infrared light). The light receiving element may receive the light reflected from the fundus Ef via a confocal aperture disposed at a position substantially conjugate with the fundus Ef.

The observation system 200 may have a configuration of a so-called fundus camera type. The OCT unit 100 may also serve as the observation system 200. That is, the fundus front image may be acquired by using tomographic image data (for example, an integrated image in a depth direction of a three-dimensional tomographic image, or an integrated value of spectral data at each XY-position).

Fixation Guide Unit

The fixation guide unit 300 has an optical system for guiding a line-of-sight direction of the eye E. The fixation guide unit 300 has a fixation target provided for the eye E, and can guide the eye E in a plurality of directions. For example, the fixation guide unit 300 has a visible light source for emitting visible light, and two-dimensionally changes a position provided with the fixation target. In this manner, the line-of-sight direction is changed, and consequently, an imaging site is changed. For example, if the fixation target is provided in a direction the same as that of an imaging optical axis, a central portion of the fundus Ef is set as the imaging site. If the fixation target is provided upward from the imaging optical axis, an upper portion of the fundus Ef is set as the imaging site. That is, the imaging site is changed depending on a position of the fixation target with respect to the imaging optical axis.

For example, as the fixation guide unit 300, it is conceivable to adopt various configurations such as a configuration of adjusting a fixation position by using a lighting position of LEDs arrayed in a matrix form and a configuration of adjusting a fixation position by controlling the lighting of the light source by causing the optical scanner to perform scanning using the light emitted from the light source. The fixation guide unit 300 may be an internal fixation lamp type or may be an external fixation lamp type.

Laser Unit

For example, the laser unit 400 oscillates the laser treatment light, and irradiates the patient's eye E with the laser light. For example, the laser unit 400 includes a laser light source 401 and a scanning unit 408. The laser light source 401 oscillates the laser treatment light (for example, a wavelength of 532 nm). For example, the scanning unit 408 includes a drive mirror and a drive unit 450. The drive unit 450 changes an angle of a reflection surface of the drive mirror.

The light emitted from the laser light source 401 is reflected on the scanning unit 408 and a dichroic mirror 30, and is focused to the fundus Ef via the objective optical system 106. At this time, an irradiation position of the laser light on the fundus Ef is changed by the scanning unit 408. The laser unit 400 may include an aiming lighting source for emitting aiming light.

Control Unit

The control unit 70 is connected to each unit of the laser treatment device 1 so as to control the overall device. For example, the control unit 70 is generally realized by a central processing unit (CPU) 71, the ROM 72, and the RAM 73. The ROM 72 stores various programs for controlling an operation of the laser treatment device, an image processing program for processing the fundus image, and an initial value. The RAM 73 temporarily stores various pieces of information. The control unit 70 may be configured to include a plurality of control units (that is, a plurality of processors).

For example, the control unit 70 acquires a light receiving signal output from the detector 120 of the OCT unit 100 and the light receiving element of the observation system 200. The control unit 70 controls the scanning unit 108 and the scanning unit 408 so as to change the irradiation position of the measurement light or the laser light. The control unit 70 controls the fixation guide unit 300 so as to change the fixation position.

The control unit 70 is electrically connected to the storage unit (for example, non-volatile memory) 72, the display unit 75, and the operation unit 76. The storage unit 74 is a non-transitory storage medium capable of holding stored content even if power is not supplied. For example, a hard disk drive, a flash ROM, and a removable USB memory can be used as the storage unit 74.

An operator inputs various operation instructions to the operation unit 76. The operation unit 76 outputs a signal in response to the input operation instruction to the control unit 70. For example, the operation unit 76 may employ at least any one user interface of a mouse, a joystick, a keyboard, and a touch panel. The control unit 70 may acquire an operation signal based on an operation of the operator which is received by the operation unit 76.

The display unit 75 may be a display mounted on a main body of the device, or may be a display connected to the main body. A personal computer (hereinafter, referred to as a "PC") may be used. A plurality of displays may be used in combination. The display unit 75 may be a touch panel. In a case where the display unit 75 is the touch panel, the display unit 75 functions as the operation unit 76. For example, the display unit 75 displays the fundus image acquired by the OCT unit 100 and the observation system 200.

The control unit 70 controls a display screen of the display unit 75. For example, the control unit 70 may output the acquired image to the display unit 75 as a still image or a moving image. The control unit 70 may cause the storage unit 74 to store the fundus image.

Control Operation

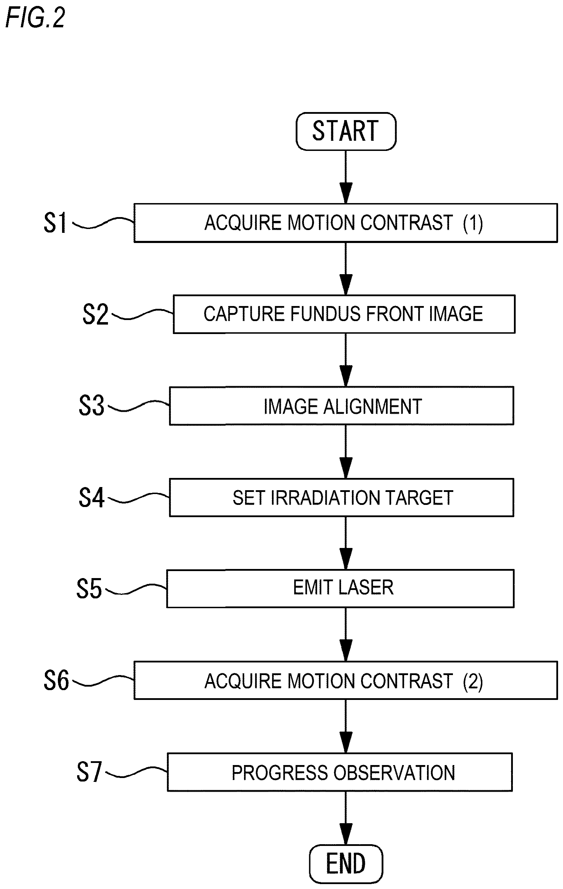

Hereinafter, a procedure when the patient's eye is treated by using the laser treatment device according to the present embodiment together with a control operation of the device will be described with reference to a flowchart in Fig. 2.

Step S1: Acquisition of Motion Contrast (1)

First, the control unit 70 acquires the motion contrast. For example, the motion contrast is information obtained by recognizing a blood flow of the patient's eye E and a change in tissues. For example, the control unit 70 may acquire the motion contrast by processing the OCT signal. In this case, the control unit 70 acquires the OCT signal by controlling the OCT unit 100.

For example, the control unit 70 controls the fixation guide unit 300 so as to provide a fixation target for a patient. Based on an anterior ocular segment observation image captured by an anterior ocular segment image capturing unit (not illustrated), the control unit 70 controls a drive unit (not illustrated) to perform automatic alignment so that the measurement light axis of the laser treatment device 1 is aligned with the center of the pupil of the patient's eye E. If the alignment is completed, the control unit 70 controls the OCT unit 100 so as to measure the patient's eye E. The control unit 70 causes the scanning unit 108 to scan the patient's eye E with the measurement light, and acquires the OCT signal of the fundus Ef.

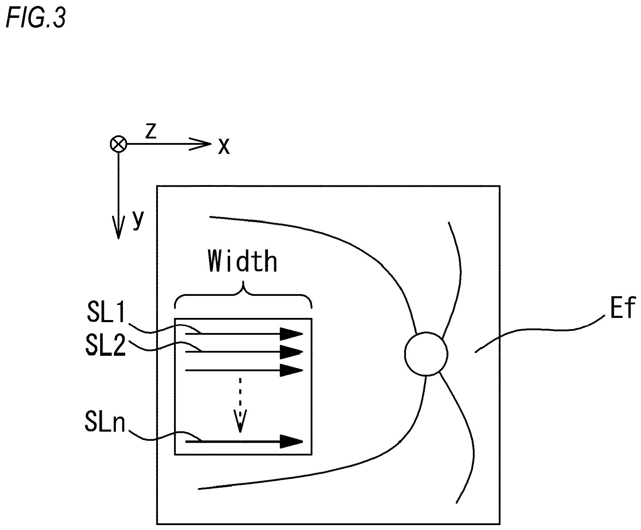

In a case where the control unit 70 acquires the motion contrast, the control unit 70 acquires at least two OCT signals which are temporally different from each other with regard to a target imaging position of the patient's eye E. For example, the control unit 70 performs scanning multiple times on the same scanning line with a predetermined time interval. For example, the control unit 70 performs first scanning on a scanning line SL1 on the fundus Ef illustrated in Fig. 3, and performs second scanning on the scanning line SL1 again after the predetermined time interval elapses. The control unit 70 acquires the OCT signal detected by the detector 120 at this time. The control unit 70 may acquire a plurality of OCT signals which are temporally different from each other with regard to the target imaging position by repeatedly performing this operation. In a case where the control unit 70 acquires the plurality of OCT signals which are temporally different from each other with regard to the target imaging position, the control unit 70 may acquire the plurality of OCT signals at the same position, or may acquire the plurality of OCT signals at positions which are slightly deviated from each other. In the present embodiment, scanning using the measurement light in a direction (for example, the X-direction) intersecting the optical axis direction of the measurement light is called "B-scan", and the OCT signal obtained by performing the B-scan once is called the OCT signal of one frame.

For example, the control unit 70 similarly acquires the plurality of OCT signals which are temporally different from each other for other scanning lines SL2 to SLn. For example, the control unit 70 acquires the plurality of OCT signals which are temporally different from each other in each scanning line, and causes the storage unit 74 to store the data.

If the OCT data is acquired, the control unit 70 acquires the motion contrast by processing the OCT data. For example, a calculation method of the OCT data for acquiring the motion contrast includes a method of calculating an intensity difference of a complex OCT signal, a method of calculating a phase difference of the complex OCT signal, a method of calculating a vector difference of the complex OCT signal, a method of multiplying the phase difference and the vector difference of the complex OCT signal, and a method of using correlation of the signals (correlation mapping). In the present embodiment, the method of calculating the phase difference for acquiring the motion contrast will be described as an example.

If the OCT signal is acquired, the control unit 70 processes the OCT signal, and acquires the motion contrast. As a calculation method of the OCT signal for acquiring the motion contrast, for example, it is conceivable to employ a method of calculating the intensity difference of the complex OCT signal, a method of calculating intensity dispersion of the complex OCT signal, a method of calculating the phase difference of the complex OCT signal, a method of calculating the vector difference of the complex OCT signal, a method of using the correlation (or decorrelation) of the OCT signal (correlation mapping or decorrelation mapping), and a method of combining the motion contrast data items obtained as described above. In the present embodiment, as an example, the method of calculating the phase difference will be described.

For example, in a case of calculating the phase difference, the control unit 70 performs the Fourier transform on the plurality of OCT signals. For example, if a signal at a position (x, z) of the N-th frame in the N-number of frames is represented by An (x, z), the control unit 70 obtains a complex OCT signal An (x, z) through the Fourier transform. The complex OCT signal An (x, z) includes a real component and an imaginary component.

The control unit 70 calculates the phase difference for the complex OCT signals An (x, z) which are acquired using at least two different times at the same position. For example, the control unit 70 uses the following expression (1), thereby calculating the phase difference. For example, the control unit 70 may calculate the phase difference in each scanning line, and may cause the storage unit 74 to store the data. An in the expression represents a signal acquired at time Tn, and * represents complex conjugate.

Expression 1

$$\Delta\Phi_n(x,z) = \arg\left(A_{n+1}(x,z) \times A_n{}^{*}(x,z)\right) \qquad (1)$$

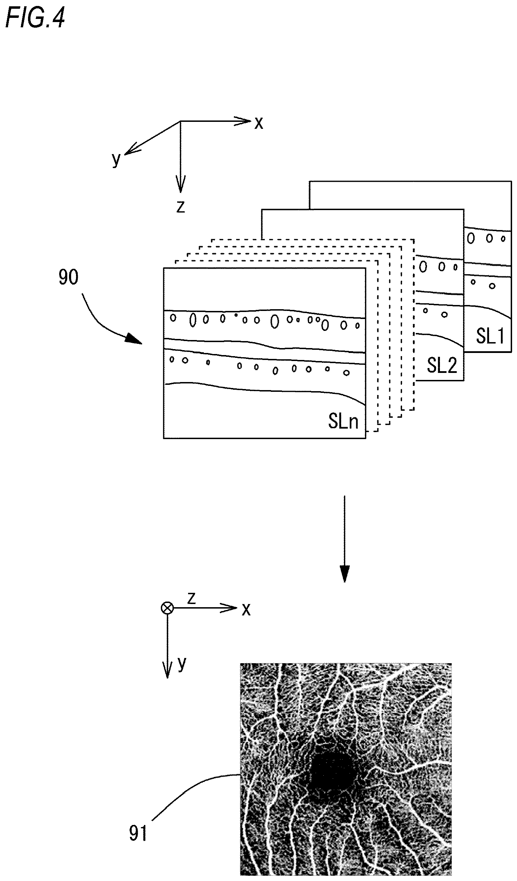

As described above, the control unit 70 acquires the motion contrast of the patient's eye E, based on the OCT data. As described above, without being limited to the phase difference, the intensity difference or the vector difference may be acquired as the motion contrast. JP-A-2015-131107 may be referred to. For example, as illustrated in Fig. 4, the control unit 70 acquires a motion contrast 90 in each scanning line.

Next, the control unit 70 generates a motion contrast front image 91 (hereinafter, abbreviated as an MC front image 91), based on the acquired motion contrast 90 (refer to Fig. 4). Here, the front image may be a so-called En face image. For example, the En face image is a plane horizontal to a fundus surface or a two-dimensional horizontal tomographic plane of a fundus.

For example, a method of generating the MC front image 91 from the motion contrast includes a method of extracting motion contrast data relating to at least a partial region in a depth direction. In this case, the MC front image 91 may be generated by using a profile of the motion contrast data in at least a partial depth region. For example, as the region in the depth direction for generating the MC front image 91, at least one of regions of the fundus Ef which are divided through segmentation processing may be selected. For example, a method of the segmentation processing includes a method of detecting a boundary of a retinal layer of the patient's eye E from a tomographic image based on the OCT signal. For example, the control unit 70 may detect the boundary of the retinal layer of the patient's eye E by detecting an edge of intensity image whose luminance value is determined in accordance with intensity of the OCT signal. For example, based on the intensity image of the patient's eye E, the control unit 70 may divide the retinal layer of the patient's eye E into a nerve fiber layer (NFL), a ganglion cell layer (GCL), a retinal pigment epithelium (RPE), and a choroid.

Since many blood vessels of the retina are present in the boundary of the retinal layer, the control unit 70 may divide a region where many blood vessels are distributed, based on the detection result of the boundary of the retinal layer. For example, a region within a predetermined range may be divided from the boundary of the retinal layer as the depth region where the blood vessels are distributed. As a matter of course, the control unit 70 may divide the depth region where the blood vessels are distributed, based on the distribution of the blood vessels detected from the motion contrast. For example, the control unit 70 may divide the region of the retina into a surface layer, an intermediate layer, and a deep layer.

Step S2: Capturing Fundus Front Image

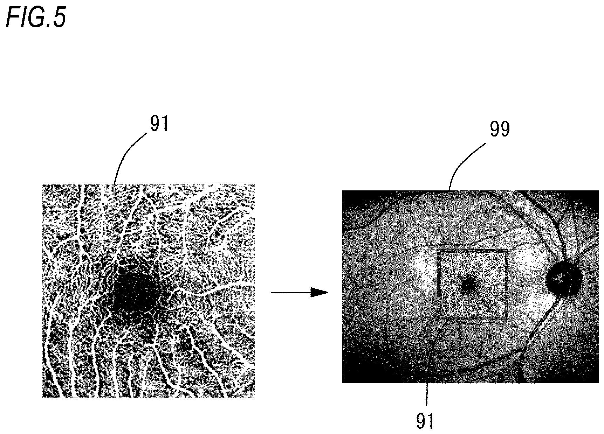

Subsequently, the control unit 70 controls the observation system 200 so as to acquire a fundus front image 99 of the patient's eye E (refer to Fig. 5). In this case, the control unit 70 acquires the fundus front image 99 so as to include at least a portion of the imaging range where the motion contrast is acquired in Step S1.

Step S3: Alignment of Image

As illustrated in Fig. 5, the control unit 70 aligns the MC front image 91 acquired in Step S1 with the fundus front image 99 acquired in Step S2. For example, the control unit 70 may align the images with each other by using various image processing methods such as a phase-only correlation method, a method of various correlation functions, a method of using the Fourier transform, a method based on feature point matching, and a method of using the affine transform.

For example, the control unit 70 may align the images with each other by displacing the MC front image 91 and the fundus front image 99 one pixel by one pixel so that both the images match each other most closely (correlation becomes highest). The control unit 70 may detect alignment information such as a displacement direction and a displacement amount of both the images. The control unit 70 may extract common features from the MC front image 91 and the fundus front image 99, and may detect the alignment information of the extracted features. For example, the control unit 70 may acquire a correspondence relationship between pixel positions of the MC front image 91 and the fundus front image 99, and may cause the memory 74 to store the correspondence relationship.

The control unit 70 may align the MC front image 91 and the fundus front image 99 with each other by using an alignment method (for example, non-rigid registration) including distortion correction. That is, the control unit 70 may align both the images after correcting image distortion between the MC front image 91 and the fundus front image 99. For example, the control unit 70 may detect image distortion information between the MC front image 91 and the fundus front image 99, and may correct the distortion of at least one image of both the images, based on the distortion information. For example, since the motion contrast needs a long measurement time, the MC front image 91 may be distorted in some cases. In a case where the MC front image 91 is distorted with respect to the fundus front image 99 in this way, characteristic regions (for example, blood vessel portions) of both images do not match each other, thereby causing a possibility that the alignment may be less likely to be performed. In this case, the control unit 70 may perform the alignment process (for example, non-rigid registration) including the distortion correction on the MC front image 91 and the fundus front image 99. In this manner, even in a case where at least a portion of the MC front image 91 is distorted, the alignment between the MC front image 91 and the fundus front image 99 can be suitably performed. As a matter of course, the distortion of the fundus front image 99 may be corrected with respect to the MC front image 91. The control unit 70 may apply the distortion information of the MC front image 91 to the whole motion contrasts which are three-dimensionally acquired. For example, the control unit 70 may develop a correction amount when the distortion correction is performed on the MC front image 91 into three-dimensional motion contrast data.

Step S4: Setting of Laser Irradiation Position (Planning)

Next, based on the motion contrast, the control unit 70 sets an irradiation target of the laser treatment light. For example, the control unit 70 sets the irradiation target, based on the MC front image 91 aligned with the fundus front image 99 in Step S3. For example, the control unit 70 causes the display unit 75 to display the MC front image 91, and causes an operator to confirm the motion contrast. In this case, the operator confirms the MC front image 91 of the display unit 75, and operates the operation unit 76, thereby selecting the irradiation target. The control unit 70 may receive an operation signal from the operation unit 76, and may set the irradiation target of the laser treatment light, based on the operation signal.

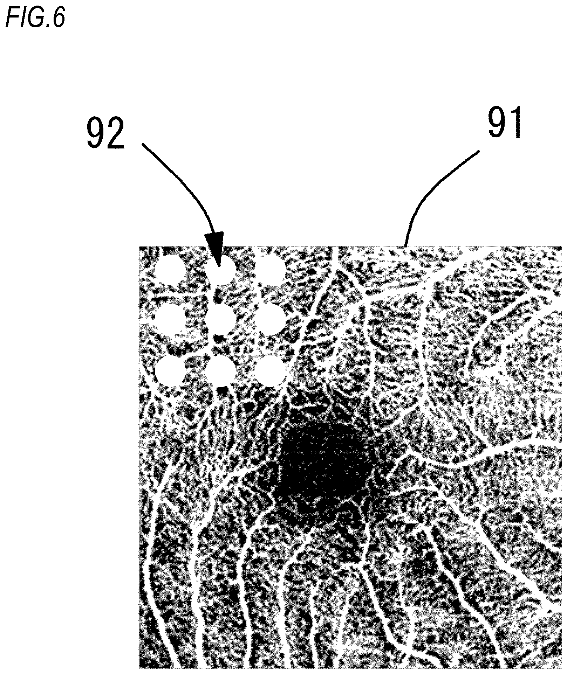

For example, as illustrated in Fig. 6, the control unit 70 causes the display unit 75 to display the MC front image 91 and an aiming mark 92 for indicating the irradiation target of the laser light. The operator moves the aiming mark 92 to a desired position while confirming a position of the blood vessel shown on the MC front image 91. For example, the operator avoids a normal blood vessel, and moves the aiming mark 92 to an affected area which is determined that laser treatment is required. In this case, the operator may move the aiming mark 92 on the MC front image 91 by using the operation unit 76. In a case where the display unit 75 is a touch panel, the operator may move the aiming mark 92 by performing a touch operation on the touch panel. The control unit 70 may move and display the position of the aiming mark 92 displayed on the MC front image 91, based on the operation signal output from the operation unit 76.

If the aiming mark 92 is moved to the desired position of the operator, for example, the control unit 70 associates the position of the aiming mark 92 on the MC front image 91 with the fundus front image 99, based on the alignment information of the MC front image 91 and the fundus front image 99. For example, the control unit 70 converts a pixel position where the aiming mark 92 is displayed on the MC front image 91 into a pixel position on the fundus front image 99. In this manner, the control unit 70 specifies the position of the aiming mark 92 on the MC front image 91 as the position on the fundus front image 99. For example, the control unit 70 sets the position selected on the MC front image 91 by the aiming mark 92 as the irradiation target of the fundus front image 99.

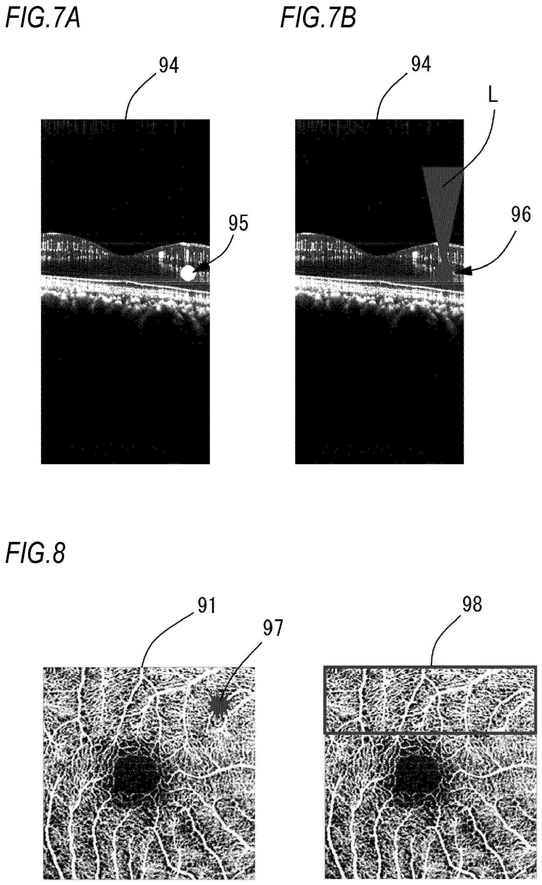

The control unit 70 may set a focal position of the laser light. For example, the control unit 70 may set the focal position of the laser light, based on the depth of the irradiation target selected by the operator. For example, the control unit 70 may cause the display unit 75 to display a motion contrast cross-sectional image (hereinafter, abbreviated as an MC cross-sectional image) 94 (refer to Fig. 7A). In this case, the operator may select a position for focusing the laser light on the MC cross-sectional image 94. The control unit 70 may display a focusing position mark 95 at the selected position on the MC cross-sectional image 94. In a case where the MC front image 91 can be displayed in a plurality of layer regions (for example, a case where the layer region of the MC front image 91 can be switched, or a case where the MC front images 91 can be simultaneously displayed in the plurality of layer regions), the control unit 70 may set the focal position of the laser light, based on the depth of the layer region of the MC front image 91 where the irradiation target is set. For example, in a case where the MC front image 91 having the set irradiation target is an image based on the motion contrast of the ganglion cell layer, the control unit 70 may set the focal position of the laser light, based on the depth of the ganglion cell layer. The control unit 70 may set the focal position of the laser light, based on the position selected by the operator. As a matter of course, when setting not only the focal position of the laser light but also the irradiation target of the laser light, the control unit 70 may use the information of the MC front images 91 in the plurality of layer regions. For example, the operator may move the aiming mark 92 while confirming the MC front images 91 in the plurality of layer regions.

Step S5: Laser Irradiation

Next, the control unit 70 controls an operation of the laser unit 400 so as to irradiate the irradiation target acquired as described above with the laser light. The control unit 70 frequently acquires the fundus front image captured by the observation system 200. The control unit 70 may cause the display unit 75 to display the fundus front image on a real time basis.

For example, if the operator operates an irradiation start key of the operation unit 76, the control unit 70 irradiates the set irradiation target with the laser light. For example, the control unit 70 controls the scanning unit 408 so as to irradiate the irradiation target with the laser light. For example, each position on the fundus front image 99 and a movable position of the scanning unit 408 are associated with each other. The control unit 70 irradiates the irradiation target on the fundus front image 99 with the laser light. In a case where a plurality of irradiation targets are present, the control unit 70 may sequentially irradiate the respective irradiation targets with the laser light.

For example, during the laser irradiation, the control unit 70 sets the fundus front image 99 associated with the MC front image 91 as a reference image for the laser light to track the irradiation target. The control unit 70 aligns the fundus front image 99 and the fundus front image 99 frequently captured by the observation system 200, and detects displacement of the patient's eye E, based on image displacement information at that time. The control unit 70 corrects the irradiation position of the laser light in accordance with the displacement (displacement of the irradiation target) of the patient's eye E. That is, in order to irradiate the set irradiation target with the laser light even if the patient's eye E is moved, the control unit 70 controls the drive of the scanning unit 408 in accordance with the detection result of the displacement. In this manner, the control unit 70 causes the irradiation position of the laser light to track the irradiation target.

The control unit 70 may adjust a focus (focal position) of the laser light in accordance with the depth of the irradiation target. For example, as illustrated in Fig. 7B, the control unit 70 may adjust a focal position 96 of laser light L in accordance with the depth of the irradiation target selected by the operator in Step S4. For example, the control unit 70 causes a drive unit 403 to move a focusing lens 402 disposed in the laser unit 400, thereby adjusting the focus of the laser light. With regard to the focus adjustment of the laser light, JP-A-2012-213634 may be referred to.

Step S6: Acquisition of Motion Contrast (2)

Subsequently, the control unit 70 acquires the motion contrast of the fundus Ef after the laser irradiation. For example, as illustrated in Fig. 8. The control unit 70 acquires a motion contrast 98 in a region including a portion if an irradiation position 97 irradiated with at least the laser light. Similarly to Step S1, the control unit 70 acquires the motion contrast of the patient's eye E.

Step S7: Progress Observation

For example, the control unit 70 may detect a change in the motion contrasts obtained before and after the laser light irradiation. For example, the motion contrast acquired in Step S1 and the motion contrast acquired in Step S2 are compared with each other. For example, the control unit 70 may obtain a difference between both the motion contrasts. For example, the control unit 70 may calculate a difference between signal strengths of the motion contrasts. For example, the control unit 70 may convert a difference value into an image, and may cause the display unit 75 to display the image. In this manner, the operator can easily confirm a state change in the patient's eye E before and after the laser irradiation.

As described above, since the motion contrast is used, it is possible to suitably perform the irradiation using the laser treatment light. For example, it is possible to perform laser treatment based on information (for example, position information of capillary blood vessels) which is less likely to be detected in observing the fundus front image or the OCT intensity image, and thus, a satisfactory treatment result can be obtained. For example, since the motion contrast is used, it is possible to acquire depth information of the blood vessel which is not recognized by a fluorescence photography image or a slit lamp. Accordingly, the control unit 70 can adjust the focus of the laser light, based on the depth information of the blood vessel. In a case where panretinal photocoagulation (PRP) is performed, the fundus is generally divided into 3 to 5 sections, and is treated at an interval of two weeks. However, a patient feels burdensome every time if the fundus is subjected to fluorescence photographing. Therefore, if the OCT unit acquires the motion contrast, both the patient and the operator can feel less burdensome.

With regard to lesions such as leakage, staining (for example, leakage of pigments due to abnormal tissues), pooling (for example, pigments leaking from a blood retinal barrier are accumulated between tissues), microaneurysm (for example, aneurysm appearing due to pressure applied to a thin artery), a blood vessel structure is less likely to be confirmed on the fluorescence photography image. Therefore, the control unit 70 may set the irradiation target of the laser light for the lesions acquired from the motion contrast image. In this manner, the irradiation position of the laser light can be aligned with the lesions which are less likely to be confirmed on the fluorescence photography image. Here, for example, the fluorescence photography is a method of imaging an eye by injecting a fluorescent agent into a patient.

The laser treatment device 1 may acquire the motion contrast from an external OCT device. For example, the laser treatment device 1 may acquire the motion contrast from the external OCT device by wireless or wired communication means. In this case, the control unit 70 may set the irradiation target of the laser light, based on the motion contrast acquired from the OCT device. The OCT device may analyze the motion contrast, and may generate setting information of the irradiation target of the laser light. The OCT device may transmit the motion contrast image and the setting information of the irradiation target to the laser treatment device 1. In this case, the laser treatment device 1 may align the motion contrast image and the fundus front image with each other, may associate the irradiation target with the fundus front image, and may irradiate the fundus Ef of the irradiation target with the laser light.

The control unit 70 may analyze the acquired motion contrast image, and may automatically set the irradiation target of the laser light by using the obtained analysis result. For example, the control unit 70 may specify a position of the lesion from the motion contrast image. The control unit 70 may set the specified lesion as the irradiation target of the laser light. For example, the control unit 70 may specify a blood leaking area or an ischemic area as the lesion. The control unit 70 may specify the blood vessel in retinal pigment epithelium (RPE) as the lesion. For example, the control unit 70 may set the blood vessel in the RPE as the irradiation target. For example, the control unit 70 may cause the display unit to display a position of a layer in the RPE. The control unit 70 may set the irradiation target of the laser light, based on shape information of a fundus layer. For example, in a case where a new blood vessel extends and the RPE is pressed up, irregularities may appear in the shape of the layer in the RPE. Therefore, the control unit 70 may set the irradiation target of the focal position of the laser light, based on the shape information of the fundus layer.

The control unit 70 may set a region determined that a state of the blood vessel is normal in the motion contrast as an irradiation prohibited region. In this manner, it is possible to avoid normal tissues from being irradiated with the laser light.

The control unit 70 may specify a predetermined area (for example, macula and papilla) of the fundus in the motion contrast through image processing, and may set the specified area as an irradiation prohibited region D. For example, the macula and the papilla may be extracted from a position, a luminance value, or a shape in the motion contrast image. Since the macular area has few blood vessels, the luminance of the macular area is darker than the luminance of the surrounding area, and the macula area has a circular shape. Accordingly, the image processing may be performed so as to extract an image region which matches the above-described characteristics. Since the papilla area has large blood vessels concentrated therein, the luminance of the papilla area is brighter than the luminance of the surrounding area, and the papilla area has a circular shape. Accordingly, the image processing may be performed so as to extract an image region which matches the above-described characteristics. As a matter of course, the control unit 70 may specify the macula and the papilla by detecting an edge. The control unit 70 may detect the macula and the papilla through the image processing by using the OCT image or the fundus front image (for example, the SLO image), and may set the specified area as the irradiation prohibited region. As a matter of course, the control unit 70 may set each position of the macula and the papilla selected by the operator from the fundus front image displayed on the display unit 75, as the irradiation prohibited region.

In the above-described tracking, as the method of tracking displacement between the two images, it is possible to employ various image processing methods (a method of using various correlation functions, a method of using the Fourier transform, or a method based on feature matching).

For example, it is conceivable to employ the following method. The reference image or the observation image (current fundus image) is displaced one pixel by one pixel, and the reference image and the target image are compared with each other, thereby detecting the displacement direction and the displacement amount between both data items when both the data items match each other most closely (correlation becomes highest). In addition, it is conceivable to employ a method of extracting common features from a predetermined reference image and target image so as to detect the displacement direction and the displacement amount between the extracted features.

As an evaluation function in template matching, the evaluation functions such as a sum of squared difference (SSD) indicating a degree of similarity and a sum of absolute difference (SAD) indicating a degree of difference may be used.

In the above-described configuration, the scanning unit is separately disposed in the OCT unit and the laser unit, but the embodiment is not limited thereto. For example, the scanning unit may be disposed on a downstream side of a point where the optical paths of the OCT unit and the laser unit are coaxial with each other. In this case, one scanning unit can perform the scanning using the measurement light emitted from the OCT unit and the laser light emitted from the laser unit.

The OCT unit and the laser unit may be configured to be respectively disposed in separate housings. For example, the irradiation target of the laser light is set in advance by using the motion contrast acquired by the OCT device, and irradiation target information thereof is input to the laser treatment device. The laser treatment device may perform the laser light irradiation, based on the input irradiation target information. The irradiation target information may be input to the laser treatment device through a communication line such as LAN. In this case, it is possible to utilize an analysis result obtained by a single OCT device. As a matter of course, the motion contrast may be acquired in such a way that the laser treatment device receives the OCT signal and analyzes the received OCT signal. The laser treatment device may receive the motion contrast from the OCT device, and may set the irradiation target, based on the received motion contrast.

As the observation system 200 disposed in the laser treatment device, a slit lamp which enables an operator to directly view images may be disposed. An in-visual field display unit may be disposed for the operator who looks into an eyepiece lens. In this case, a beam combiner is disposed between the eyepiece lens of the slit lamp and the patient's eye. A display image displayed on the in-visual field display unit is reflected on the beam combiner, and is transmitted toward the eyepiece lens. In this manner, the operator visibly recognizes the observation image and the display image of the slit lamp.

In this case, the control unit 70 may cause the in-visual field display unit to display the analysis result acquired as described above, and may display the fundus observation image and the motion contrast image by superimposing both of these on each other. In this case, the operator can set the irradiation target of the laser light with reference to the motion contrast image while viewing the fundus image.

In the above-described configuration, a configuration in which the OCT device acquires the motion contrast in the fundus and irradiates the fundus with the laser light has been described as an example, but the embodiment is not limited thereto. Any configuration may be adopted as long as the OCT device acquires the motion contrast of the eye and irradiates the tissues of the eye with the laser light, based on the acquired motion contrast. For example, a configuration may also be adopted in which the OCT device acquires the motion contrast of the motion contrast of an anterior ocular segment and irradiates the anterior ocular segment with the laser light, based on the acquired motion contrast.

The control unit 70 may acquire the motion contrast in a plurality of regions of the fundus. Furthermore, the control unit 70 may generate a panorama motion contrast image of the fundus by combining the motion contrasts acquired in the plurality of regions. In this case, the control unit 70 may align the panorama motion contrast image with a panorama fundus front image captured by the observation system 200, and may perform the laser light irradiation at a position of the panorama fundus front image corresponding to the irradiation target set on the panorama motion contrast image.

Based on the motion contrast, the control unit 70 may acquire vascular density information of the fundus. For example, the vascular density is obtained using a ratio of a region corresponding to the blood vessel per unit area in the motion contrast. For example, the control unit 70 may cause the display unit to display a density map image indicating the vascular density. For example, the density map image may be a color map image displayed using color classification according to the vascular density. For example, as the vascular density becomes higher, the density map image has the color classification so that the colors are gradually changed in the order of blue, green, yellow, and red colors. As a matter of course, without being limited to the above-described color classification, other colors may be used for the density map image.

For example, an operator may confirm the density map image, and may set an ischemic area (for example, a region having low vascular density) as the irradiation target of the laser light. The blood does not flow in the ischemic area, and cells thereof are in an acid deficient state. Accordingly, a new blood vessel extends in order to supply oxygen. In the new blood vessel, blood components are likely to leak, thereby adversely affecting a visual function. Therefore, the ischemic area is irradiated with the laser light so as to kill the cells. In this manner, the oxygen does not need to be supplied to the cells, thereby restraining the new blood vessel from being generated. The operator can easily confirm the ischemic area by using the density map image of the blood vessel, and comfortably set the irradiation target.

The control unit 70 may automatically perform the laser light irradiation, based on the vascular density information. For example, the control unit 70 may set the ischemic area obtained from the vascular density information as the irradiation target, and may cause the laser unit 400 to irradiate the ischemic area with the laser light. In this way, the laser light irradiation is automatically performed using the vascular density information. Therefore, the labor of the operator for setting the irradiation target of the laser light can be saved, and the laser light irradiation can be performed at a suitable position.

**Claims**

1. An ophthalmic laser treatment device comprising:

   an irradiation unit configured to irradiate a patient's eye with laser treatment light; and
   control means for:

      acquiring a motion contrast acquired by an OCT unit configured to detect an OCT signal of measurement light reflected from the patient's eye and reference light corresponding to the measurement light;
      acquire irradiation target information based on the motion contrast; and
      control the irradiation unit to irradiate the patient's eye with the laser light based on the irradiation target information.

2. The ophthalmic laser treatment device according to claim 1 further comprising:

   an image capturing unit configured to capture a fundus front image of the patient's eye,
   wherein the control means aligns an image of the motion contrast and the fundus front image with each other, and irradiates an irradiation target of which the irradiation target information is associated with the fundus front image, with the laser light.

3. The ophthalmic laser treatment device according to claim 2, wherein the control means detects displacement of the irradiation target which occurs due to a motion of the patient's eye, from the fundus front images which are frequently captured by the image capturing unit, and causes an irradiation position of the laser light to track the irradiation target based on the detected displacement.

**4.** The ophthalmic laser treatment device according to claim 1, wherein the control means controls a focal position of the laser light, based on the irradiation target information.

**5.** The ophthalmic laser treatment device according to claim 1,
wherein the control means acquires, from the OCT unit, the motion contrasts in a region including at least an irradiation position of the laser light used for irradiation based on the irradiation target information, and compares the motion contrast acquired before the laser light irradiation and the motion contrast acquired after the laser light irradiation with each other.

**6.** An ophthalmic laser treatment system comprising:

an ophthalmic laser treatment device configured to irradiate a patient's eye with laser treatment light; and
an OCT device configured to detect an OCT signal of measurement light reflected from the patient's eye and reference light corresponding to the measurement light,
wherein the OCT device calculates a motion contrast, based on the OCT signal, and
wherein the ophthalmic laser treatment device acquires irradiation target information based on the motion contrast, and irradiates the patient's eye with the laser light, based on the irradiation target information.

**7.** A method of controlling an ophthalmic laser treatment device, the method comprising steps of:

acquiring a motion contrast acquired by an OCT unit that detects an OCT signal of measurement light reflected from a patient's eye and reference light corresponding to the measurement light;
acquiring irradiation target information based on the motion contrast; and
irradiating the patient's eye with laser treatment light based on the irradiation target information.

FIG.1

*FIG.2*

```
                    ┌──────────┐
                    │  START   │
                    └────┬─────┘
                         │
                         ▼
S1  ┌──────────────────────────────────────────┐
    │      ACQUIRE MOTION CONTRAST  (1)         │
    └──────────────────┬───────────────────────┘
                       │
                       ▼
S2  ┌──────────────────────────────────────────┐
    │        CAPTURE FUNDUS FRONT IMAGE         │
    └──────────────────┬───────────────────────┘
                       │
                       ▼
S3  ┌──────────────────────────────────────────┐
    │             IMAGE ALIGNMENT              │
    └──────────────────┬───────────────────────┘
                       │
                       ▼
S4  ┌──────────────────────────────────────────┐
    │          SET IRRADIATION TARGET          │
    └──────────────────┬───────────────────────┘
                       │
                       ▼
S5  ┌──────────────────────────────────────────┐
    │               EMIT LASER                 │
    └──────────────────┬───────────────────────┘
                       │
                       ▼
S6  ┌──────────────────────────────────────────┐
    │      ACQUIRE MOTION CONTRAST  (2)         │
    └──────────────────┬───────────────────────┘
                       │
                       ▼
S7  ┌──────────────────────────────────────────┐
    │           PROGRESS OBSERVATION           │
    └──────────────────┬───────────────────────┘
                       │
                       ▼
                    ┌──────────┐
                    │   END    │
                    └──────────┘
```

FIG.3

## FIG.4

FIG.5

91

99

91

FIG.6

*FIG.7A*

94

95

*FIG.7B*

94

L

96

*FIG.8*

91

97

98

**EP 3 213 670 A1**

Europäisches
Patentamt

European
Patent Office

Office européen
des brevets

# EUROPEAN SEARCH REPORT

Application Number

EP 17 15 8832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/238046 A1 (DICK MANFRED [DE] ET AL) 29 September 2011 (2011-09-29) * abstract * * paragraphs [0013] - [0019], [0037] - [0040] * | 1,4-7 | INV. A61B3/12 A61F9/008 |
| X | DE 10 2007 005699 A1 (ZEISS CARL MEDITEC AG [DE]) 7 August 2008 (2008-08-07) * abstract * * paragraphs [0053], [0075] * * claim 17 * | 1,2,4-7 | |
| X | EP 2 468 225 A1 (NIDEK KK [JP]) 27 June 2012 (2012-06-27) * abstract * * paragraphs [0012], [0045], [0057], [0063] - [0065] * | 1-7 | |
| X | WO 2016/011043 A1 (UNIV ROCHESTER [US]) 21 January 2016 (2016-01-21) * abstract * * page 9, line 9 - page 10, line 12 * | 1,4-7 | TECHNICAL FIELDS SEARCHED (IPC) |
| X | US 2013/261612 A1 (YOKOSUKA HIROKI [JP] ET AL) 3 October 2013 (2013-10-03) * paragraphs [0023], [0047], [0064] * | 1,2,4,6,7 | A61B A61F |

The present search report has been drawn up for all claims

| Place of search | Date of completion of the search | Examiner |
|---|---|---|
| Munich | 10 July 2017 | Jansen, Birte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 213 670 A1**

## ANNEX TO THE EUROPEAN SEARCH REPORT
## ON EUROPEAN PATENT APPLICATION NO.

EP 17 15 8832

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2017

| Patent document cited in search report | | Publication date | Patent family member(s) | | Publication date |
|---|---|---|---|---|---|
| US 2011238046 | A1 | 29-09-2011 | DE 102010012810 A1<br>US    2011238046 A1 | | 29-09-2011<br>29-09-2011 |
| DE 102007005699 | A1 | 07-08-2008 | DE 102007005699 A1<br>EP       2117488 A1<br>US    2010145319 A1<br>WO    2008095659 A1 | | 07-08-2008<br>18-11-2009<br>10-06-2010<br>14-08-2008 |
| EP 2468225 | A1 | 27-06-2012 | EP       2468225 A1<br>JP       5842330 B2<br>JP    2012135550 A<br>US    2012165799 A1<br>US    2016302969 A1 | | 27-06-2012<br>13-01-2016<br>19-07-2012<br>28-06-2012<br>20-10-2016 |
| WO 2016011043 | A1 | 21-01-2016 | US    2017189228 A1<br>WO    2016011043 A1 | | 06-07-2017<br>21-01-2016 |
| US 2013261612 | A1 | 03-10-2013 | JP       5958027 B2<br>JP    2012213634 A<br>US    2013261612 A1 | | 27-07-2016<br>08-11-2012<br>03-10-2013 |

EPO FORM P0459

For more details about this annex : see Official Journal of the European Patent Office, No. 12/82

**EP 3 213 670 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010148635 A
- JP 2008029467 A
- JP 2015131107 A
- JP 2012213634 A